(19) Europäisches Patentamt

European Patent Office

Office européen des brevets

(11) **EP 1 637 879 A1**

(12) **EUROPÄISCHE PATENTANMELDUNG**

(43) Veröffentlichungstag:
**22.03.2006 Patentblatt 2006/12**

(51) Int Cl.:
*G01N 33/28* (2006.01)  *G01N 33/00* (2006.01)

(21) Anmeldenummer: **04022471.9**

(22) Anmeldetag: **21.09.2004**

(84) Benannte Vertragsstaaten:
**AT BE BG CH CY CZ DE DK EE ES FI FR GB GR HU IE IT LI LU MC NL PL PT RO SE SI SK TR**
Benannte Erstreckungsstaaten:
**AL HR LT LV MK**

(71) Anmelder: **EMH Energie-Messtechnik GmbH**
**21438 Brackel (DE)**

(72) Erfinder: **Schröder, Karsten**
**20146 Hamburg (DE)**

(74) Vertreter: **Seemann, Ralph**
**Patentanwälte Seemann & Partner,**
**Ballindamm 3**
**20095 Hamburg (DE)**

(54) **Verfahren und Vorrichtung zur quantitativen Analyse von Gasen und/oder Ölfeuchte in einem Transformatoröl**

(57) Die Erfindung betrifft ein Verfahren und eine Vorrichtung zur quantitativen Analyse von Gasen in einem Transformatoröl. Das erfindungsgemäße Verfahren zeichnet sich durch die folgenden Verfahrensschritte aus:

- Trennen eines Gases vom Transformatoröl und Einbringen des Gases in eine Messkammer (11),

- Messen der Konzentration wenigstens einer Gaskomponente im Gas, wobei für die Bestimmung der Konzentration der wenigstens einen Gaskomponente zwei Sensoren (16, 17) Verwendung finden.

Die erfindungsgemäße Vorrichtung umfasst eine Verbindungsvorrichtung (13, 15), die zwischen einem Anschlusselement (27) und einer Messkammer (11) angeordnet ist, wobei durch die Verbindungsvorrichtung (13, 15) Transformatoröl strömbar ist, wobei ein gasdurchlässiges und Öl zurückhaltendes Trennelement (14, 41) vorgesehen ist, das an die Verbindungsvorrichtung (13) und die Messkammer (11) angrenzt, wobei zur Messung der Konzentration einer Gaskomponente zwei Sensoren (16, 17) in der Messkammer (11) vorgesehen sind.

Fig. 1

**Beschreibung**

Die Erfindung betrifft ein Verfahren und eine Vorrichtung zur quantitativen Analyse von Gasen und/oder Ölfeuchte in einem Transformatoröl.

Transformatoren gehören zu den wichtigsten und kostenintensivsten Betriebsmitteln der elektrischen Energieversorgung, weswegen die auftretenden Fehler in diesen Komponenten nicht nur zur Unterbrechung der elektrischen Energieversorgung führen, sondern zu wirtschaftlichen Schäden. Ein Transformatorbetrieb soll eine kontinuierliche Energieversorgung möglichst über Jahre hinweg fehlerfrei sichern. Aus diesem Grund ist es gewünscht, dass auftretende Fehler, die eventuell Ausfälle der Transformatoren verursachen können, rechtzeitig erkannt werden, um geeignete Maßnahmen zur Fehlerbeseitigung einzuleiten. Eine kontinuierliche Erfassung des Isolationszustandes kann somit große Fehler verhindern, die Nutzungsdauer eines Transformators verlängern und die Instandhaltung optimieren.

Als Isoliermittel wird in Leistungstransformatoren eine Kombination aus einem flüssigen und einem festen Isolierstoff eingesetzt. Fehler in der Isolation von flüssigkeitsgefüllten Transformatoren sind fast immer von der Entwicklung in Öl gelöster Gase sowie einer Erhöhung der Ölfeuchte begleitet. Die Menge und Zusammensetzung der Zersetzungsgase hängt sowohl von der Isolierflüssigkeit als auch von der Art und der Energiemenge des zugrunde liegenden Fehlers ab. Große Fehler mit hohem Energiegehalt verursachen voluminöse Gasmengen in kurzer Zeit, während die produzierten Gase bei kleinen Fehlern verhältnismäßig gering sind. Ein Grund für die Entstehung dieser Gase ist die Zersetzung fester und flüssiger Isolierstoffe, die durch Teilentladungen und Kreisströme, örtliche Überhitzungen durch Kurzschlusswindungen, hohe Übergangswiderstände oder starke Wirbelströme sowie durch Lichtbogenentladungen und - überschläge hervorgerufen werden können. Dabei erfolgt durch elektrische und/oder thermische Energiezufuhr eine Zerstörung der langkettigen Ölmoleküle, wodurch insbesondere Wasserstoff und leichte Kohlenwasserstoffverbindungen entstehen. Bei der Zersetzung von Zellulose entsteht zusätzlich Kohlenmonoxid und Kohlendioxid. Je nach der Menge der entstandenen Gase können sie in gelöster und/oder ungelöster Form auftreten. Bei entsprechend aufgespaltenen Molekülen können so auch Wassermoleküle gebildet werden, die zu einer unerwünschten Ölfeuchte führen.

Die derzeit im Handel befindlichen Messsysteme für in einem Transformatoröl gelöste bzw. ungelöste Gase sind nur eingeschränkt nutzbar. Hierzu sei beispielsweise auf die US 5 773 709 A verwiesen. In diesem Dokument ist eine Vorrichtung zur Überwachung einer Substanz in einer dielektrischen Flüssigkeit bekannt, wobei diese Flüssigkeit im Inneren eines elektrischen Systems vorgesehen ist. Es sind Verbindungsmittel vorgesehen, um die Flüssigkeit zu einem Trennmittel zu leiten, wobei das Trennmittel vorgesehen ist, um die Flüssigkeit und ein Gas, das in der Flüssigkeit angeordnet ist, zu trennen, um das Gas anschließend zu messen bzw. zu analysieren. Zur besseren Gasübergabe sind Wärmemittel vorgesehen, mittels denen eine Flüssigkeitskonvektion im Verbindungsmittel erzeugt wird, so dass sich die Flüssigkeit am Trennmittel entlang bewegt.

Es ist Aufgabe der vorliegenden Erfindung, ein effizientes, sicheres und möglichst genaues Verfahren zur quantitativen Analyse von Gasen in einem Transformatoröl als auch eine entsprechende Vorrichtung hierzu anzugeben.

Gelöst wird diese Aufgabe durch ein Verfahren zur quantitativen Analyse von Gasen in einem Transformatoröl umfassend die folgenden Verfahrensschritte:

- Trennen eines Gases vom Transformatoröl und Einbringen des Gases in eine Messkammer,

- Messen der Konzentration wenigstens einer Gaskomponente im Gas, wobei für die Bestimmung der Konzentration der wenigstens einen Gaskomponente zwei Sensoren Verwendung finden.

Durch das erfindungsgemäße Verfahren kann sehr effizient und genau festgestellt werden, ob sich ein vorbestimmbares bzw. vorgebbares Gas im Transformatoröl befindet, das von der Art bzw. der Menge her sicherheitsrelevant werden könnte. Durch Verwendung von zwei Sensoren für diese Gaskomponenten erhöht sich die Genauigkeit der Bestimmung der Menge des Gases.

Wenn die Sensoren gleich sind, kann bei der Analyse des zu messenden Gases bzw. der Gaskomponente der Mittelwert der beiden Messwerte herangezogen werden, so dass die Messung genauer wird. Außerdem kann hierdurch im System festgestellt werden, ob sich beide Sensoren richtig verhalten oder ob einer der beiden Sensoren wegdriftet. Hierdurch wird die Verlässlichkeit des erfindungsgemäßen Verfahrens deutlich erhöht.

Vorzugsweise sind die Sensoren Wasserstoffsensoren. In diesem Fall ist die zu bestimmende Gaskomponente Wasserstoff ($H_2$).

Wenn eine weitere Gaskomponente mit zwei weiteren, insbesondere gleichen, Sensoren gemessen wird, können im wesentlichen alle relevanten auftretenden Fehler in einem Transformator, der mit einem flüssigen Dielektrikum wie Öl gefüllt ist, herausgefunden werden. Vorzugsweise ist die weitere Gaskomponente Kohlenmonoxid (CO). Mit der Detektion bzw. quantitativen Analyse der Gaskomponenten Wasserstoff und Kohlenmonoxid in dem Transformatoröl können überraschenderweise im Wesentlichen sämtliche Probleme, die mit Transformatoren auftreten, detektiert werden. Als weitere Gase können zwar auch $C_2H_2$ und $C_2H_4$ sowie weitere brennbare Gase entstehen; diese treten allerdings, wie erfindungsgemäß festgestellt wurde, nicht ohne Wasserstoff oder Kohlenmonoxid auf, so dass es an sich schon ausreichend wäre, nur die Menge von Wasserstoff zu messen, wobei es auf jeden Fall ausreichend ist, sowohl Wasserstoff als auch Kohlenmonoxid zu messen.

Der Sensor bzw. die Sensoren, die für das Kohlenmonoxid Verwendung finden, haben eine entsprechende Querempfindlichkeit insbesondere zu Wasserstoff. Als Sensor für Wasserstoff findet der Sensor TGS 821 der Firma Figaro USA, Inc. vorzugsweise Verwendung und zum Messen des Gasbestandteils Kohlenmonoxid ein Sensor TGS 203 der Firma Figaro. Es können noch weitere Sensoren Verwendung finden, die beispielsweise besonders sensitiv sind für $C_2H_2$ und $C_2H_4$, für den Gesamtgehalt brennbarer Kohlenwasserstoffverbindungen $C_xH_x$ oder weitere Gase. Im Falle von $C_2H_2$ kann beispielsweise der Sensor TGS 261 der Firma Figaro Verwendung finden. Der letztgenannte Sensor hat allerdings eine entsprechende Querempfindlichkeit zu Wasserstoff und Kohlenmonoxid, so dass dieser Sensor erfindungsgemäß insbesondere dann Verwendung findet, wenn auch je ein bzw. zwei Wasserstoff- und Kohlenmonoxidsensoren vorhanden sind und hiermit die entsprechenden Wasserstoff- und Kohlenmonoxidkonzentrationen bestimmt werden.

Die Lebensdauer insbesondere der Wasserstoffsensoren wird dann deutlich erhöht, wenn diese in vorgebbaren Perioden geheizt und insbesondere das Heizen der Sensoren zeitweise unterbrochen wird. Dieses ist insbesondere beim Wasserstoffsensor unüblich, da bei diesem im Betriebshandbuch vorgesehen ist, dass der Sensor dauernd geheizt wird, so dass ununterbrochen die Wasserstoffkonzentration gemessen wird. Diese Vorgabe ergibt sich insbesondere aus dem Einsatz in der Gaswarntechnik, bei der eine lückenlose Überwachung erforderlich ist. Hiervon wird gemäß der Erfindung abgegangen. Stattdessen wird in entsprechenden Perioden, also in vorgebbaren Zeitintervallen, wie unter Bezugnahme auf die Zeichnung noch näher beschrieben wird, geheizt und gemessen.

Die Lebensdauer der Sensoren wird außerdem dadurch deutlich erhöht, dass die Betriebsspannung wenigstens eines Sensors kleiner als die Hälfte, insbesondere kleiner als ¼ und insbesondere kleiner als 1/10 der vorgesehenen maximalen Betriebsspannung ist. Hierdurch wird insbesondere sichergestellt, dass auch bei sehr hohen Gaskonzentrationen nicht die maximal mögliche Leistung des Sensors überstiegen wird. Die im Rahmen dieser Anmeldung beispielsweise verwendeten Gassensoren sind Festkörpersensoren, die einen kleineren Widerstand aufweisen, je größer die Konzentration der Gaskomponente ist. Bei entsprechend vorgebbarer Betriebsspannung und entsprechend hoher Konzentration wird dann ein entsprechend hoher Strom erzeugt, der durch den Sensor fließt. Durch Verringern der Betriebsspannung wird verhindert, dass der Strom zu groß wird. So wird beispielsweise der TGS 821 anstelle mit einer Betriebsspannung von 24 V mit 1 V betrieben und der TGS 203 anstelle von 5 V mit 1 V betrieben.

Wenn die Messkammer eine Öffnung zur Umgebung aufweist, ist die Genauigkeit der Bestimmung der Konzentration der Gaskomponenten erhöht.

Durch Verwendung von jeweils zwei Sensoren je Gaskomponente wird das Ergebnis der quantitativen Bestimmung der Menge bzw. Konzentration der Gaskomponente im Gas durch entsprechendes Mitteln der Messwerte deutlich verbessert. Außerdem kann, wie oben schon dargestellt wurde, ein Fehler eines Sensors leichter festgestellt werden.

Vorzugsweise hängt die Konzentration des Wasserstoffs, die mit dem Wasserstoffsensor gemessen wird, von einer Summe ab, deren Terme verschiedene Potenzen des Widerstandes des Sensors aufweisen. Die Konzentration von Wasserstoff ist hierbei vorzugsweise nach der folgenden Formel gegeben:

$$C_{H2} = k1 + k2 * R + \frac{k3}{R^{1,5}} \qquad (1)$$

Wenn der additive Term

$$\frac{k4 * \ln R}{R^2}$$

hinzugefügt wird, erhöht sich die Genauigkeit der Konzentrationsbestimmung. Diese verbessert sich weiter, wenn der additive Term

$$\frac{k5}{R^2}$$

hinzugefügt wird. Hierbei ist R der Widerstand des Wasserstoffgassensors, $C_{H2}$ die Konzentration von Wasserstoff in ppm und k1 bis k5 Konstanten, wobei k1 im Bereich von -10 bis +10 liegt, k2 im Bereich von $1 \times 10^{-6}$ bis $1 \times 10^{-3}$, k3 im Bereich von $1 \times 10^{8}$ bis $1 \times 10^{9}$, k4 im Bereich von $-1 \times 10^{9}$ bis $-5 \times 10^{9}$ und k5 im Bereich von $4 \times 10^{9}$ bis $10^{10}$.

Die Konzentration von Kohlenmonoxid ist eine Summe mit Summanden, die Potenzen des Widerstands des Kohlenmonoxidsensors aufweisen und Potenzen der eben dargestellten Konzentration von Wasserstoff. Erfindungsgemäß ergibt sich die Kohlenmonoxidkonzentration nach der folgenden Formel

$$C_{CO}{}^{-1} = k6 + k7 \cdot R^{1,5} + k8 \cdot C_{H2}{}^{0,5} \qquad (2),$$

wobei $C_{CO}$ die Konzentration von Kohlenmonoxid in ppm ist und k6 eine Konstante zwischen -0,5 und +0,5, k7 eine Konstante im Bereich von 1 bis $5 \times 10^{-7}$ und k8 eine Konstante im Bereich von 1 bis $5 \times 10^{-6}$.

Die Konzentration weiterer Gase wie beispielsweise $C_2H_2$ ergibt sich durch Messung an wenigstens einem weiteren Sensor, vorzugsweise zwei weiteren Sensoren, wie beispielsweise dem TGS 2610 gemessen. Dieser Sensor hat Querempfindlichkeiten zu Wasserstoff und Kohlenmonoxid. Die bevorzugte Variante sieht dann insgesamt 6 Sensoren vor. Bei Kenntnis der Konzentrationen von Wasserstoff und Kohlenmonoxid kann die Konzentration von $C_2H_2$ bestimmt werden nach einer Summenformel, die Terme aufweist, die quadratische Anteile der Konzentrationen von Kohlenmonoxid und Wasserstoff aufweisen sowie vorzugsweise Exponentialfunktionen, wobei die Wasserstoff- und Kohlenmonoxidkonzentrationen im Exponenten stehen. Vorzugsweise kann die Summenformel einen weiteren Term aufweisen, in der die Quadrate der Konzentrationen von Wasserstoff und Kohlenmonoxid miteinander multipliziert werden. Die Quadrate dieser Konzentrationen stehen vorzugsweise im Nenner des Terms.

Die Aufgabe wird ferner durch eine Vorrichtung zur quantitativen Analyse von Gasen in einem Transformatoröl, umfassend eine Verbindungsvorrichtung, die zwischen einem Anschlusselement und einer Messkammer angeordnet ist, gelöst, wobei durch die Verbindungsvorrichtung Transformatoröl strömbar ist, wobei ein gasdurchlässiges und Öl zurückhaltendes Trennelement vorgesehen ist, das an die Verbindungsvorrichtung und die Messkammer angrenzt, wobei zur Messung der Konzentration und vorzugsweise auch zur Analyse einer Gaskomponente zwei Sensoren in der Messkammer vorgesehen sind. Vorzugsweise sind die Sensoren gleich. Weiter vorzugsweise sind diese Sensoren Wasserstoffsensoren. Werden zwei weitere, insbesondere gleiche, Sensoren für eine weitere Gaskomponente, insbesondere für Kohlenmonoxid, vorgesehen, ist eine besonders effiziente Vorrichtung gegeben.

Die Messkammer weist vorzugsweise eine Öffnung zur Umgebung auf. Diese ist vorzugsweise vergleichsweise klein. Der Durchmesser kann beispielsweise in einem Bereich von 0,5 bis 2 mm, insbesondere bei 1 mm liegen.

Besonders effizient ist die Vorrichtung, wenn die Verbindungsvorrichtung rohrförmig ausgebildet ist. Wenn der lichte Durchmesser der Verbindungsvorrichtung im Bereich des Trennelements aufgeweitet ist, ist eine genauere quantitative Analyse der Gase bzw. Gaskomponenten möglich.

Vorzugsweise umfasst das Trennelement Polytetrafluorethylen. Das Trennelement umfasst vorzugsweise eine Membran, ferner umfasst das Trennelement vorzugsweise eine Sintermetallscheibe, die an der Membran angeordnet ist. Die Sintermetallscheibe dient hierzu beispielsweise als Stützelement für die Membran, die Polytetrafluorethylen umfassen kann. Die Sintermetallscheibe ist beispielsweise eine CrNi-Sintermetallscheibe mit einer Dicke von ca. 3 mm und einer Porengröße von ca. 0,5 $\mu$m.

Die Erfindung wird nachstehend ohne Beschränkung des allgemeinen Erfindungsgedankens anhand von Ausführungsbeispielen unter Bezugnahme auf die Zeichnungen beschrieben. Bezüglich aller im Text nicht näher erläuterten erfindungsgemäßen Einzelheiten wird ausdrücklich auf die Zeichnungen verwiesen.

Es zeigen:

Fig.1     eine schematische teilweise transparente Darstellung einer erfindungsgemäßen Analysevorrichtung,

Fig. 2     eine erfindungsgemäße Analysevorrichtung in dreidimensionaler Darstellung, wobei ein Teil des Gehäuses entfernt wurde,

Fig. 3     eine Kurve der Heizspannung über die Zeit,

Fig. 4     eine weitere Kurve der Heizspannung über die Zeit

Fig. 5     eine schematische Darstellung eines erfindungsgemäßen Monitoringsystems,

Fig. 6    eine schematische Schnittdarstellung der Anbringung eines Feuchtesensors,

Fig. 7    eine schematische Seitenansicht einer erfindungsgemäßen Analysevorrichtung in auseinander gebautem Zustand und

Fig. 8    eine Schaltung zum Betreiben des Feuchtesensors.

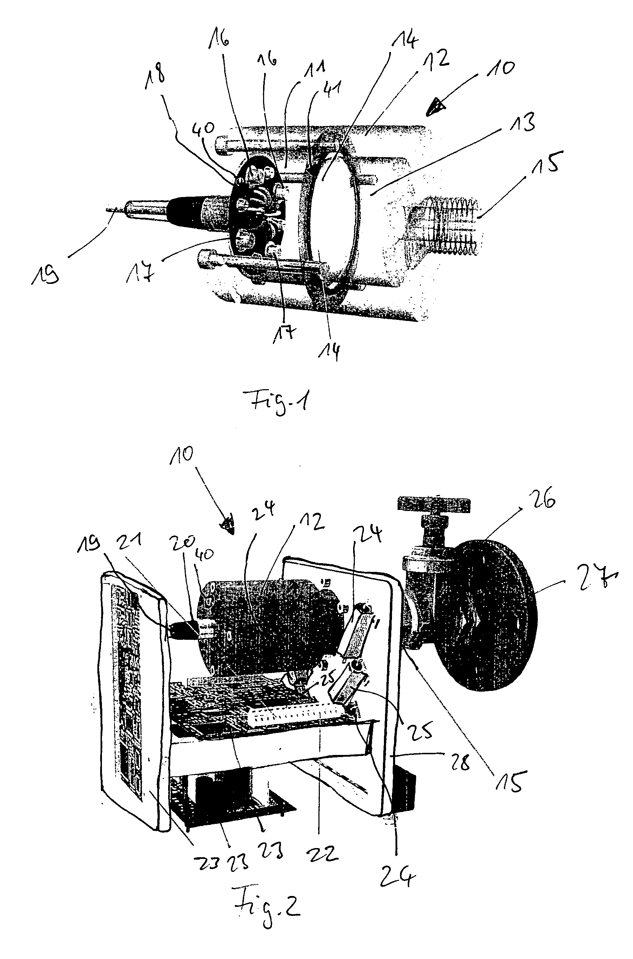

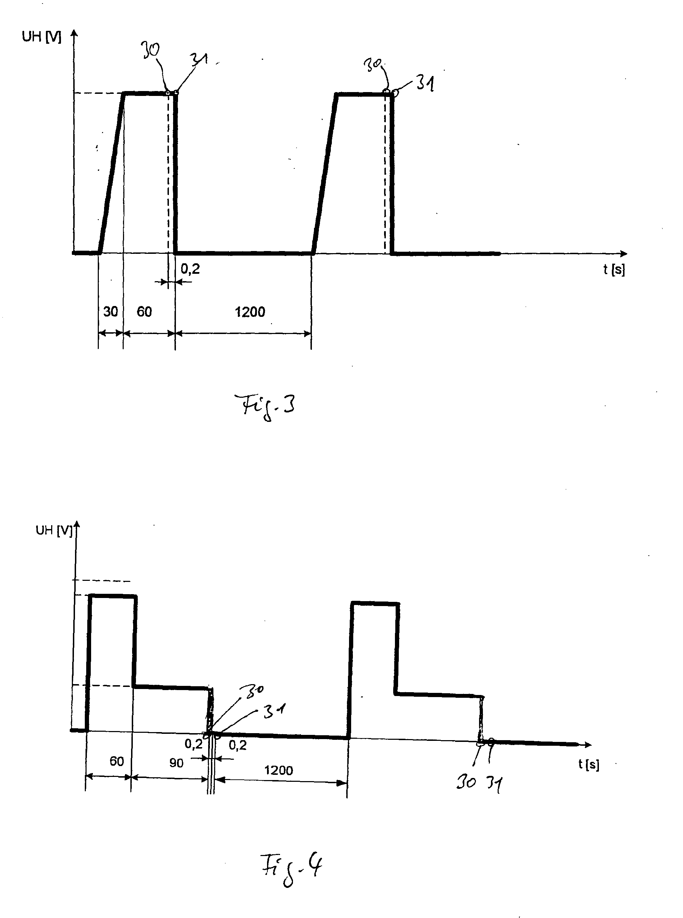

Fig. 1 zeigt teilweise transparent dargestellt eine erfindungsgemäße Analysevorrichtung 10. Die Analysevorrichtung 10 umfasst einen Trennkorpus 12, der eine Messkammer 11 und einen Ölraum 13 aufweist. Der Ölraum 13 und die Messkammer 11 sind durch eine Membran 14 getrennt. Die Membran 14 kann Polytetrafluorethylen sein. Dieses ist unter der Marke Teflon® bekannt. Die Membran 14 kann, muss aber nicht unbedingt, durch eine Sintermetallscheibe 41 gestützt sein, damit bei zu großen Druckunterschieden zwischen dem Ölraum 13 und der Messkammer 11 die Membran 14 nicht reißt. Öl gelangt über ein Rohr 15 in den Ölraum 13. Damit ein entsprechender Ölfluss entsteht, ist beispielsweise vorgesehen, die Umgebung der Analysevorrichtung 10 und insbesondere eine Gehäuseseite auf der Seite des Ölraums 13 zu heizen. Hierzu wird in Bezugnahme auf die Fig. 2 näher eingegangen werden.

Etwaiges in Öl gelöstes bzw. ungelöstes Gas gelangt über die Membran 14 in die Messkammer 11, wohingegen Öl aufgrund der Membran 14 im Ölraum 13 zurückgehalten wird. Das Rohr 15 ist in Verbindung mit beispielsweise einem Ölkreislauf eines Transformators. Hierzu ist, wie in Fig. 2 dargestellt, ein Flansch 27 vorgesehen, der entsprechend an einem Transformator angeflanscht werden kann und ein Ventil 26, mittels dieses die Verbindung zu dem Transformator hergestellt werden kann.

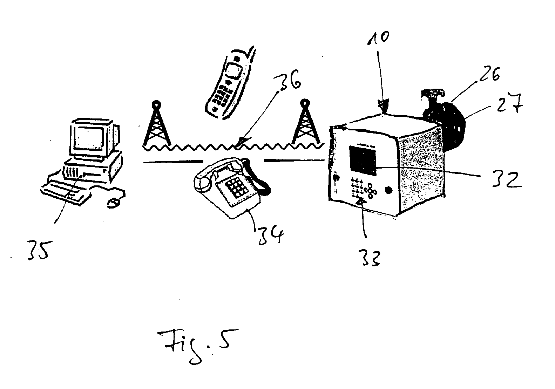

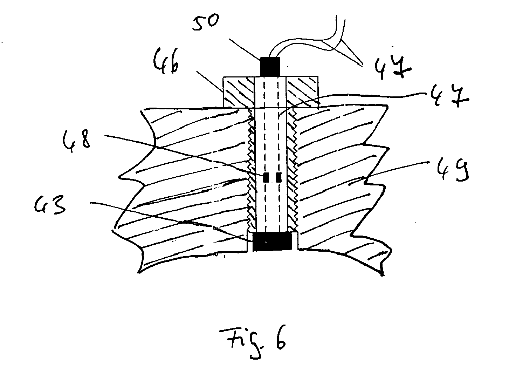

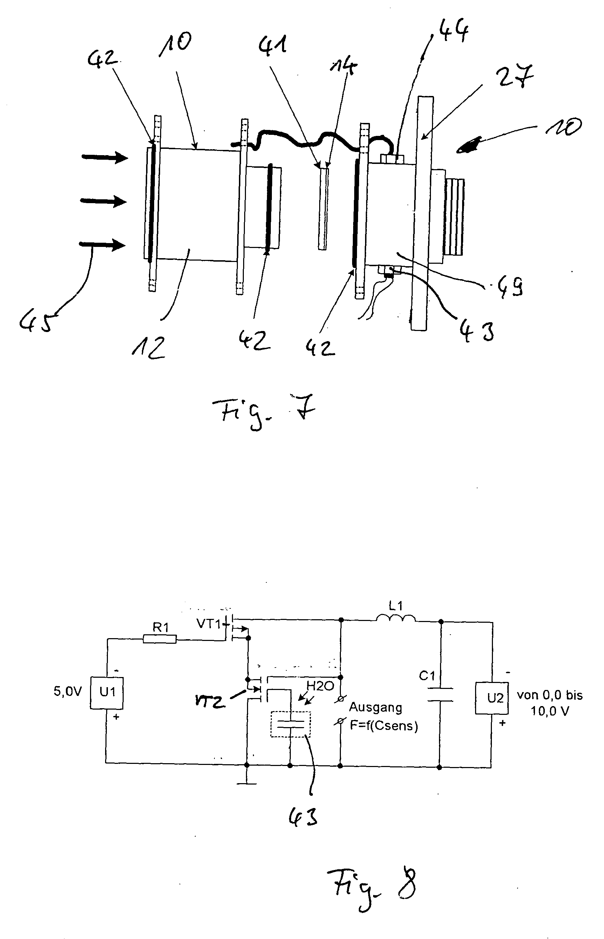

Es sind ferner vier Sensoren vorgesehen, und zwar jeweils zwei Sensoren 16 und zwei Sensoren 17, die in diesem Ausführungsbeispiel zur Detektion von Wasserstoff, beispielsweise durch die beiden Sensoren 16, und zur Detektion von Kohlenmonoxid, beispielsweise durch die beiden Sensoren 17 ausgebildet sind. Entsprechende Kabel 18 sind mit den jeweiligen PINs der Sensoren verbunden. Bei den Sensoren handelt es sich um Festkörpersensoren, beispielsweise die Sensoren TGS 821 und TGS 203 der Firma Figaro USA, Inc. Bei diesen Sensoren handelt es sich um Festkörpersensoren aus einem halbleitenden Metalloxid, wie beispielsweise im Fall des TGS 821 um Zinndioxid ($SnO_2$). Die Leitfähigkeit ist an Luft relativ gering. Diese erhöht sich bei Vorhandensein der entsprechenden Gase, wie Wasserstoff oder Kohlenmonoxid. Es sind jeweils zwei Kabel vorgesehen, um die Sensoren entsprechend aufzuheizen, um die Messtemperatur zu erreichen, und zwei Kabel, um die Betriebsspannung anzulegen, um entsprechend den Strom messen zu können, so dass der jeweilige Widerstand der Sensoren bestimmt werden kann. Die Kabel 18 werden durch die Leitung 19 und einen Stecker 20 zu einer Analyseelektronik weitergeleitet. Die Analyseelektronik ist in Fig. 2 dargestellt. Die hier dargestellte Systemelektronik 23 umfasst mehrere Platinen, auf denen entsprechende Bauelemente angeordnet sind. Es ist außerdem vorzugsweise auch eine CPU (Prozessoreinheit) vorgesehen, um entsprechende Berechnungen durchzuführen. Mit einer digitalen Schnittstelle 21 und einer analogen Schnittstelle 22 wird eine Verbindung nach außen hergestellt. Die Verbindung nach außen wird unter Bezugnahme auf die Fig. 5 näher erläutert.

In Fig. 2 sind auch Heizwiderstände 24 und Peltierelemente 25 dargestellt, wobei die Heizwiderstände 24 dazu dienen, einen Bereich der Analysevorrichtung 10 aufzuheizen und die Peltierelemente 25 dazu dienen, diesen Bereich abzukühlen. Das Aufheizen dient dazu, die Diffusionskoeffizienten der zu bestimmenden Gase, wie Wasserstoff und Kohlenmonoxid, aus dem Öl reproduzierbar konstant zu halten. Hierbei soll versucht werden, die Temperatur des Öls und die Temperatur des Flansches 27 bzw. die Temperatur im Rohr 15 bzw. im Ölraum 13 unterschiedlich zur Öltemperatur im Transformator vorzusehen, da ansonsten kein Ölaustausch stattfindet. Um einen ausreichenden Ölaustausch vorzusehen, ist vorgesehen, einen Temperaturzyklus zu erzeugen, der eine Flanschtemperatur von 35 °C +/- 5 °C vorsieht und eine Zeitperiode von 1 - 3 Stunden, vorzugsweise 2 Stunden. Sofern die Öltemperatur kleiner als die Außentemperatur ist, ist vorgesehen, den Temperaturzyklus bei 45 °C +/- 5 °C vorzusehen. Auch hierbei ist die gleiche Zeitperiode sinnvoll. Der Zyklus kann sinusförmig, dreieckförmig oder anders ausgestaltet sein. Um den Temperaturgradienten zwischen dem Öl und dem Flansch zu erhöhen, ist es auch vorgesehen, den Flansch bzw. die Rückwand des Gehäuses mittels einiger Peltierelemente 25 abzukühlen. Auch hierzu kann abwechselnd mit dem Aufheizen durch die Heizwiderstände 24 ein Temperaturzyklus gefahren werden.

Im Gegensatz zur Angabe im Datenblatt zum TGS 121 wird, wie in Fig. 3 dargestellt ist, das Beheizen des Sensors periodisch durchgeführt. Fig. 3 zeigt eine derartige Kurve der Heizspannung über der Zeit. Die numerischen Angaben in Fig. 3 und in Fig. 4 sind in Sekunden. Zunächst wird über 30 Sekunden der Sensor durch eine Erhöhung der Heizspannung auf eine maximale Höhe aufgewärmt. Das entsprechende Heizen findet in einer Periode von 60 Sekunden statt. Am Ende dieser Periode, d. h. nach 58,8 Sekunden, wird der erste Messwert genommen. 0,2 Sekunden später der zweite Messwert. Anschließend wird sofort die Heizspannung für 1200 Sekunden auf 0 V heruntergefahren, um mit dem Messzyklus von vorne zu beginnen. Dieses Heizschema wird beim Wasserstoffsensor vorzugsweise verwendet. Durch das periodische Heizen wird erreicht, dass die Lebensdauer des Sensors deutlich erhöht wird.

Zur Messung der Konzentration des jeweiligen Gasbestandteils (bspw. Wasserstoff) bzw. in diesem Fall zunächst des Widerstandes der Sensoren, um daraus die Konzentration des Wasserstoffes festzustellen, ist zu sagen, dass die Widerstandsmessung bei einer Betriebsspannung von 1 V durchgeführt wird, also bei einer deutlich kleineren Betriebsspannung als die maximal angegebene Betriebsspannung von 24 V. Auch hierdurch erhöht sich die Lebensdauer des Sensors erheblich.

Kohlenmonoxid wird mit einem Sensor (bzw. zwei Sensoren) mit einem Heizschema, wie in Fig. 4 dargestellt ist, gemessen. Zunächst wird die Heizspannung auf einen relativ hohen Wert eingestellt, um nach 60 Sekunden auf ca. 1/3 dieses Wertes heruntergeregelt zu werden. Diese Spannung bleibt für 90 Sekunden aufrechterhalten. Anschließend wird die Spannung auf 0 V geregelt und sofort der erste Messwert 30 genommen und anschließend nach 0,2 Sekunden der Messwert 31. Die Spannung bleibt für 1200 Sekunden auf 0 V um den Heiz- und Messzyklus von neuem zu beginnen. Auch hier dient eine Betriebsspannung von 1 V anstelle von 5 V, wie diese im Datenblatt angegeben ist, dazu, die Lebensdauer des Sensors deutlich zu erhöhen. Beim Sensor, der gemäß Fig. 4 geheizt wird, handelt es sich vorzugsweise um den TGS 203.

Es ist auch möglich, aber erfindungsgemäß nicht notwendig, auch weitere Gaskomponenten zu messen. Hierzu werden dann weitere Sensoren verwendet, wie beispielsweise ein $C_2H_2$-Sensor oder eine $C_2H_4$-Sensor. Diese Sensoren, wie beispielsweise ein TGS 2620 der Figaro US, Inc., haben entsprechend Querempfindlichkeiten zu Kohlenmonoxid und Wasserstoff. Aus diesem Grunde ist auch immer der Kohlenmonoxid- und Wasserstoffgehalt in der Messzelle zu messen. Die Messzelle selbst ist vorzugsweise relativ klein, beispielsweise mit einem Volumen von 40 bis 100 ml, vorzugsweise 60 ml. Die Öffnung in der Messzelle ist vorzugsweise im Durchmesser ca. 1 mm, um einen quasi geschlossenen Raum und keinen zu großen Austausch der Gase im Raum mit der Umgebung zu ermöglichen.

Das erfindungsgemäße Verfahren und die erfindungsgemäße Vorrichtung eignen sich für ein Monitoring der Gase in einem Dielektrikum, insbesondere Öl eines Transformators, wobei, wie in Fig. 5 dargestellt ist, eine Datenfernübermittlung vorgesehen ist. Die Datenfernübermittlung kann über eine Telefonverbindung 34 zu einer Überwachungsvorrichtung 35 geschehen oder über eine Funkvorrichtung 36, wie beispielsweise mittels UMTS oder GSM. Hierzu ist das mobile Telefon angedeutet. Die entsprechenden Messwerte, die auf dem Display 32 auch in Grafikform, somit auch in zeitlicher Veränderung, dargestellt werden können, wozu eine Bedientafel 33 vorgesehen ist, um die gewünschten Parameter einzustellen oder das gewünschte Gas auszuwählen, werden über die Datenfernübermittlung zu einer Überwachungsvorrichtung 35 gesendet. Die entsprechenden Messwerte können dann auch über die Datenfernverbindung der Überwachungsvorrichtung 35 zugeführt werden, wobei beispielsweise die Messdaten übermittelt werden können und in der Überwachungsvorrichtung 35 selbst aus den Messwerten entsprechende Überwachungskurven bzw. -funktionen realisiert werden können. Es können allerdings auch aktualisierte Kurven der Gasbestandteile, bei denen die Konzentration der Gasbestandteile über die Zeit aufgetragen sind, übermittelt werden.

Durch dieses Gas-in-Öl- bzw. Gasmonitoringsystem ist es möglich, mehrere Transformatoren an einem Überwachungsort zu überwachen und rechtzeitig Maßnahmen einzuleiten, wie beispielsweise Wartungen zu initiieren oder Fehlerbehebungen an entsprechenden Transformatoren. Dieses führt zu einer sehr hohen Kosteneffizienz. Beispielsweise können analoge Signale ausgegeben werden, wie die Wasserstoffkonzentration und/oder die Kohlenmonoxidkonzentration. Hierzu dienen die analogen Ausgänge 22, die in Fig. 2 dargestellt sind. Außerdem können Alarme digital ausgegeben werden, wie ein Wasserstoffalarm oder ein Kohlenmonoxidalarm, wobei verschiedene Alarmniveaus Verwendung finden können, beispielsweise ein Alarm, der bei einer mittleren Konzentration ausgegeben wird, und ein Alarm, der eine massive Gefährdung der Anlage anzeigt. Es können am digitalen Ausgang 21, der in Fig. 2 dargestellt ist, auch Betriebsfehler ausgegeben werden. Die Alarmniveaukonfigurationen können einstellbar sein. Es können auch weitere Messwerte, wie Öltemperatur oder die Umgebungstemperatur, weitergegeben werden. Es kann auch ein Wegdriften eines Sensors als Alarm weitergegeben werden. Schließlich kann ein Kühlsystem und können weitere Kenngrößen eines Transformators überwacht werden.

Es kann ferner auch wichtig sein, den Wassergehalt des Isolieröls zu bestimmen. Hierzu wird vorzugsweise ein kapazitiver Feuchtesensor mit der Bezeichnung Humicape K der Firma Vaisala verwendet.

Fig. 6 zeigt eine schematische Schnittdarstellung eines Teils eines Rohrgehäuses 49, in der eine entsprechende Schraube 46 eingeschraubt ist, auf die bzw. in die ein Feuchtesensor 43 eingebracht ist. Der Feuchtesensor 43 ist in Kontakt mit dem Öl im Ölraum 13. Hierzu ist eine entsprechende Schraube 46, beispielsweise eine Sechskantschraube, aufgebohrt, um die elektrischen Verbindungen des Feuchtesensors 43 mit entsprechenden Kabeln 47 an entsprechenden Anschlussstellen 48 vorzunehmen. Der entstehende Hohlraum wird beispielsweise durch ein Harz oder einen Bikomponentenkleber flüssigkeitsdicht und auch gegen entsprechende Drücke, die im Ölraum 13 vorherrschen, abgedichtet. Zur sicheren mechanischen Verbindung ist außerdem ein Schrumpfschlauch 50 vorgesehen, der die Kabel 47 mechanisch fixiert.

Fig. 7 zeigt eine schematische Seitenansicht einer Analysevorrichtung 10 in auseinander gebautem Zustand. In der Analysevorrichtung 10, die aus zwei Trennkorpussen 12 besteht, die in entsprechend zusammengebautem Zustand dafür sorgen, dass die Membran 14, die auf eine Sintermetallscheibe 41 aufgebracht ist, eine Abdichtung der Messkammer 11 gegenüber dem Ölraum 13 ermöglicht, zeigt unter anderem auch den Einbau des Feuchtesensors 43 in einem Trennkorpus bzw. in dem Rohrgehäuse 49. In diesem Rohrgehäuse 49 ist auch eine Temperaturmessvorrichtung 44, wie beispielsweise ein PT 100, vorgesehen. Es sind außerdem in Fig. 7 drei Dichtungsringe 42, wie beispielsweise O-Ringe, dargestellt, die zur Abdichtung der jeweiligen Komponenten und Kammern dienen. Es ist ferner eine Einbaurichtung 45 durch die angedeuteten Pfeile dargestellt.

Der verwendete Feuchtesensor ist auf Basis einer Dünnschichttechnologie hergestellt. Auf einem Glassubstrat aus beispielsweise BrSi sind zwei Elektroden aus Gold oder Chrom oder eine Legierung hieraus aufgebracht. Als Dielektrikum dient Aluminiumoxid, beispielsweise ein Polymer wie z.B. Tetraethoxylan, Hexamethyldisiloxan oder Zellulose-Acetat. Auch $AL_2O_3$ kann als Dielektrikum dienen. Dieses Dielektrikum hat eine hohe Feuchtempfindlichkeit im Hinblick auf dessen Dielektrizitätskonstante. Eine aufgebrachte Deckelelektrode besteht beispielsweise aus Gold, das in einer solchen Schichtdicke bzw. Schichtart aufgedampft ist, dass diese noch durchlässig für Wassermoleküle ist, aber keine großflächigen Löcher auf der Oberfläche hinterlässt. Es wird eine entsprechende Sensorkapazität, die zwischen den beiden Metallelektroden entsteht, über ein elektrisches Feld im Dielektrikum unterhalb der Gegenelektrode gekoppelt. Näherungsweise kann die Kapazität des Sensors in Form eines Plattenkondensators betrachtet werden, wobei die Variation der relativen Dielektrizitätskonstante ausgenutzt wird. Es gilt dann, die folgende Form

$$C_{H2O} = \varepsilon_0 {}^* \varepsilon_{H2O} {}^* (A/d) \qquad (3)$$

Hierbei ist $\varepsilon_{H2O}$ die Dielektrizitätskonstante des Wassers, $\varepsilon_0$ die Vakuumdielektrizitätskonstante, A die aktive Oberfläche der feuchteempfindlichen Schicht des Sensors und d die Dicke des Dielektrikums. Wenn Wassermoleküle durch die poröse Goldschicht defundieren und auf der Oberfläche der feuchteempfindlichen Schicht absorbiert oder adsorbiert werden, ändert sich entsprechend die Dielektrizität dieser Schicht. Die Menge des absorbierenden Wassers und damit die Kapazität des Sensors sind vom Wassergehalt im Isolieröl und damit ist die Kapazität des Sensors vom Wassergehalt im Isolieröl und der Temperatur des Isolieröls abhängig.

Um den feuchtigkeitsempfindlichen Sensor zu beschalten, wird eine Schaltung gemäß Fig. 8 verwendet. Hierzu ist es möglich, astabile Multivibratorschaltungen, kapazitive Brückenschaltungen und Frequenzumsetzungsschaltungen zu verwenden. In Fig. 8 ist eine Frequenzumsetzschaltung auf Basis von zwei komplementären MOSFETs VT1 und VT2 realisiert. Es wird ein Oszillatorschwingkreis gebildet, und zwar mittels der Außeninduktivität L1 und der Innenkapazität der komplementären MOSFETs VT1 und VT2 mit einem negativen Widerstand, der an den Elektroden "Drain-Source" von VT1 und "Drain-Gate" von VT2 gebildet werden. Es wird eine Volt-Ampere bzw. Spannungs-Strom-Charakteristik mit negativem Widerstand realisiert. Vorzugsweise liegt der Arbeitspunkt der Schaltung auf dem abfallenden Bereich der Charakteristik, in dem eine Oszillation entsteht. Der Feuchtesensor liegt am Gate des MOSFET VT2 an. Durch die Änderung der Kapazität des Feuchtesensors ändert sich die Innenkapazität des Oszillatorschwingkreises und entsprechend die Resonanzfrequenz der Schaltung. So entsteht eine Abhängigkeit der Resonanzfrequenz der Schaltung von der Feuchte bzw. des Wassers im Öl, die mit der folgenden Formel beschrieben werden kann:

$$F(a_w) = 1/2\pi \sqrt{LC_{sensor} {}^* \varepsilon}_{(H2O+Öl)} \qquad (4)$$

wobei $\varepsilon_{(H2o+Öl)} = a_w (\varepsilon_{H2O} - \varepsilon_{Öl}) + \varepsilon_{Öl}$ ist und wobei $\varepsilon_{H2O}$ im Bereich von 80 liegt und $E_{Öl}$ im Bereich von 2,3. Die Sensorkapazität wird wie folgt bestimmt:

$$C_{sens} = C_0 {}^* [a_w {}^* (\varepsilon_{H2O} + \varepsilon_{Öl})] \qquad (5)$$

wobei $C_0$ die Anfangskapazität des Sensors in der Luft ist. Außerdem wurde eine Temperaturabhängigkeit durch eine Korrektur wie folgt eingeführt:

$$a_W = a_{W\ 20°C} + a {}^* \Delta T \qquad (6).$$

Hierbei ist $\Delta$ der Temperaturkoeffizient im Temperaturbereich von 20°C bis 40°C und entspricht im Wesentlichen 0,33%/°C. Außerdem ist $a_W$ 20°C die Wasseraktivität bei 20°C und $\Delta T$ die Abweichung der Temperatur von 20°C.

Die Wasseraktivität definiert den aktiven Teil des im Isolieröl vorhandenen Wassers. Öl kann Wasser in drei verschiedenen Phasen enthalten, je nach Wassermenge und Öltemperatur gelöst, frei und emulgiert. Schmieröle und Isolieröle haben einen gewissen Gehalt an gelöstem Wasser. Eine einfache Möglichkeit den Wassergehalt festzustellen, besteht in der Messung der Wasseraktivität $a_W$ des Öls. Dieser ist ein relativer Wert zur Angabe der prozentualen Sättigung des Öls und ist dabei unabhängig von Alter, Temperatur und chemischer Zusammensetzung. Die Sättigung des Öls mit Wasser ist umgekehrt proportional zur Öltemperatur. Sinkt die Temperatur plötzlich, besteht akute Gefahr, dass sich freies Wasser abscheidet. Mit dem erfindungsgemäßen Online-Sensor zur Überwachung der Wasseraktivität wird sofort ein Messwert ermittelt und ein Alarm gemeldet, wenn die Gefahr besteht, dass Wasser als Phase abscheiden kann. Freies Wasser führt nämlich ausnahmslos zu einem Abfall der elektrischen Festigkeit und des elektrischen Widerstandes sowie zu einem Anstieg des dielektrischen Verlustfaktors, was bei Transformatoren naturgemäß unerwünscht ist.

Bezugszeichenliste

| | |
|---|---|
| 10 | Analysevorrichtung |
| 11 | Messkammer |
| 12 | Trennkorpus |
| 13 | Ölraum |
| 14 | Membran |
| 15 | Rohr |
| 16 | erster Sensor |
| 17 | zweiter Sensor |
| 18 | Kabel |
| 19 | Leitung |
| 20 | Stecker |
| 21 | digitale Schnittstelle |
| 22 | analoge Schnittstelle |
| 23 | Systemelektronik |
| 24 | Heizwiderstand |
| 25 | Peltierelement |
| 26 | Ventil |
| 27 | Flansch |
| 28 | Gehäuse |
| 30 | erster Messpunkt |
| 31 | weiter Messpunkt |
| 32 | Display |
| 33 | Bedientafel |
| 34 | Telefonverbindung |
| 35 | Überwachungsvorrichtung |
| 36 | Funkverbindung |
| 40 | Öffnung |
| 41 | Sintermetallscheibe |
| 42 | Dichtungsring |
| 43 | Feuchtesensor |
| 44 | Temperaturmessvorrichtung |
| 45 | Einbaurichtung |
| 46 | Schraube |
| 47 | Kabel |
| 48 | Anschlussstelle |
| 49 | Rohrgehäuse |
| 50 | Schrumpfschlauch |
| VT1 | MOSFET |
| VT2 | MOSFET |
| U1 | Versorgungsstrang |

U2 variabler Strang
R1 Widerstand
L1 Spule
C1 Kondensator

**Patentansprüche**

1.  Verfahren und Vorrichtung zur quantitativen Analyse von Gasen in einem Transformatoröl, umfassend die folgenden Verfahrensschritte:

    - Trennen eines Gases vom Transformatoröl und Einbringen des Gases in eine Messkammer (11),
    - Messen der Konzentration wenigstens einer Gaskomponente im Gas, wobei für die Bestimmung der Konzentration der wenigstens einen Gaskomponente zwei Sensoren (16, 17) Verwendung finden.

2.  Verfahren nach Anspruch 1, **dadurch gekennzeichnet, dass** die Sensoren (16, 17) gleich sind.

3.  Verfahren nach Anspruch 1 oder 2, **dadurch gekennzeichnet, dass** die Sensoren (16, 17) Wasserstoffsensoren sind.

4.  Verfahren nach einem oder mehreren der Ansprüche 1 bis 3, **dadurch gekennzeichnet, dass** eine weitere Gaskomponente mit zwei weiteren, insbesondere gleichen, Sensoren (16, 17) gemessen wird.

5.  Verfahren nach Anspruch 4, **dadurch gekennzeichnet, dass** die weitere Gaskomponente Kohlenmonoxid ist.

6.  Verfahren nach einem oder mehreren der Ansprüche 1 bis 5, **dadurch gekennzeichnet, dass** die Sensoren (16, 17), insbesondere die Wasserstoffsensoren, in vorgebbaren Perioden geheizt werden.

7.  Verfahren nach Anspruch 6, **dadurch gekennzeichnet, dass** das Heizen der Sensoren (16, 17) zeitweise unterbrochen wird.

8.  Verfahren nach einem oder mehreren der Ansprüche 1 bis 7, **dadurch gekennzeichnet, dass** die Betriebsspannung wenigstens eines Sensors (16, 17) kleiner als die Hälfte, insbesondere kleiner als ¼ und insbesondere kleiner als 1/10, der vorgesehenen maximalen Betriebsspannung ist.

9.  Verfahren nach einem oder mehreren der Ansprüche 1 bis 8, **dadurch gekennzeichnet, dass** die Messkammer (11) eine Öffnung (40) zur Umgebung aufweist.

10. Vorrichtung zur quantitativen Analyse von Gasen in einem Transformatoröl, umfassend eine Verbindungsvorrichtung (13, 15), die zwischen einem Anschlusselement (27) und einer Messkammer (11) angeordnet ist, wobei durch die Verbindungsvorrichtung (13, 15) Transformatoröl strömbar ist, wobei ein gasdurchlässiges und Öl zurückhaltendes Trennelement (14, 41) vorgesehen ist, das an die Verbindungsvorrichtung (13) und die Messkammer (11) angrenzt, wobei zur Messung der Konzentration einer Gaskomponente zwei Sensoren (16, 17) in der Messkammer (11) vorgesehen sind.

11. Vorrichtung nach Anspruch 10, **dadurch gekennzeichnet, dass** die Sensoren (16, 17) gleich sind.

12. Vorrichtung nach Anspruch 10 oder 11, **dadurch gekennzeichnet, dass** die Sensoren (16, 17) Wasserstoffsensoren sind.

13. Vorrichtung nach einem oder mehreren der Ansprüche 10 bis 12, **dadurch gekennzeichnet, dass** zwei weitere, insbesondere gleiche, Sensoren (16, 17) für eine weitere Gaskomponente, insbesondere für Kohlenmonoxid vorgesehen sind.

14. Vorrichtung nach einem oder mehreren der Ansprüche 10 bis 13, **dadurch gekennzeichnet, dass** die Messkammer (11) eine Öffnung (40) zur Umgebung aufweist.

15. Vorrichtung nach einem oder mehreren der Ansprüche 10 bis 14, **dadurch gekennzeichnet, dass** die Verbindungsvorrichtung (13, 15) rohrförmig ausgebildet ist.

**16.** Vorrichtung nach Anspruch 15, **dadurch gekennzeichnet, dass** der lichte Durchmesser der Verbindungsvorrichtung (13, 15) im Bereich des Trennelements (14) aufgeweitet ist.

**17.** Vorrichtung nach einem oder mehreren der Ansprüche 10 bis 16, **dadurch gekennzeichnet, dass** das Trennelement (14, 41) Polytetrafluorethylen umfasst.

**18.** Vorrichtung nach einem oder mehreren der Ansprüche 10 bis 17, **dadurch gekennzeichnet, dass** das Trennelement (14, 41) eine Membran (14) umfasst.

**19.** Vorrichtung nach Anspruch 18, **dadurch gekennzeichnet, dass** an der Membran (14) eine Sintermetallscheibe angeordnet ist.

Fig. 1

Fig. 2

Fig. 3

Fig. 4

Fig. 5

Fig. 6

42    10    41    14    44    27    10

45    43    12    42    42    43    49

Fig. 7

R1    VT1    L1

5,0V    U1    VT2    H2O    C1    U2    von 0,0 bis
                              Ausgang              10,0 V
                              F=f(Csens)

43

Fig. 8

**Europäisches Patentamt**

**EUROPÄISCHER RECHERCHENBERICHT**

**Nummer der Anmeldung**

EP 04 02 2471

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int.Cl.7) |
|---|---|---|---|
| Y | BIRLASEKARAN S ET AL: "Use of FFT and ANN techniques in monitoring of transformer fault gases" PROCEEDINGS OF 1998 INTERNATIONAL SYMPOSIUM ON ELECTRICAL INSULATING MATERIALS, 27-30 SEPT. 1998, TOYOHASHI, JAPAN, 27. September 1998 (1998-09-27), Seiten 75-78, XP002318699 ISBN: 4-88686-050-8 * Zusammenfassung; Abbildung 1 * * Seite 76, rechte Spalte, Absatz 1; Abbildung 4 * ----- | 1-19 | G01N33/28 G01N33/00 |
| Y | DE 103 33 323 A1 (CONTINENTAL TEVES AG & CO. OHG) 29. Januar 2004 (2004-01-29) * Zusammenfassung * * Seite 6, Absatz 34 * ----- | 1-19 | |
| A | DD 238 116 A1 (ORGREB-INSTITUT FUER KRAFTWERKE,DD) 6. August 1986 (1986-08-06) * Zusammenfassung; Abbildung 1 * ----- | 1-19 | **RECHERCHIERTE SACHGEBIETE (Int.Cl.7)** |
| A | DE 101 58 746 A1 (SIEMENS AG) 18. Juni 2003 (2003-06-18) * Zusammenfassung; Abbildungen 4,5,8,9 * * Spalte 4, Absatz 32 * ----- | 9,14,15, 18,19 | G01N |
| Y | BIRLASEKARAN S ET AL: "Development of a carbon monoxide gas-in-oil detector for transformers" PROCEEDINGS OF THE IPEC '95 - INTERNATIONAL POWER ENGINEERING CONFERENCE 1995, 27 FEB. - 1 MARCH 1995, SINGAPORE, Bd. 1, 27. Februar 1995 (1995-02-27), Seiten 346-348, XP009044341 * Zusammenfassung; Abbildung 1 * * Seite 346, rechte Spalte, Absatz 2 * ----- | 1-19 | |

Der vorliegende Recherchenbericht wurde für alle Patentansprüche erstellt

| Recherchenort | Abschlußdatum der Recherche | Prüfer |
|---|---|---|
| München | 22. Februar 2005 | Hanisch, C |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument
........................................................................
& : Mitglied der gleichen Patentfamilie, übereinstimmendes Dokument

EPO FORM 1503 03.82 (P04C03)

**ANHANG ZUM EUROPÄISCHEN RECHERCHENBERICHT
ÜBER DIE EUROPÄISCHE PATENTANMELDUNG NR.**

EP 04 02 2471

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

22-02-2005

| Im Recherchenbericht angeführtes Patentdokument | Datum der Veröffentlichung | Mitglied(er) der Patentfamilie | Datum der Veröffentlichung |
|---|---|---|---|
| DE 10333323 A1 | 29-01-2004 | KEINE | |
| DD 238116 A1 | 06-08-1986 | KEINE | |
| DE 10158746 A1 | 18-06-2003 | WO 03048719 A2 | 12-06-2003 |
| | | EP 1448985 A2 | 25-08-2004 |

EPO FORM P0461

Für nähere Einzelheiten zu diesem Anhang : siehe Amtsblatt des Europäischen Patentamts, Nr.12/82